# EUROPÄISCHE PATENTSCHRIFT

(11) **EP 1 098 627 B1**
(45) Veröffentlichungstag und Bekanntmachung des Hinweises auf die Patenterteilung: **28.01.2004**
(21) Anmeldenummer: 99929194.1
(22) Anmeldetag: 12.06.1999
(51) Int. Cl.: A61K 7/13

(54) **FÄRBEMITTEL ZUM FÄRBEN VON KERATINFASERN**
COLORANT FOR COLOURING KERATIN FIBRES
COLORANT POUR COLORER DES FIBRES KERATINIQUES

(30) Priorität: 23.06.1998 DE 19827000; 23.07.1998 US 93926 P
(43) Veröffentlichungstag der Anmeldung: 16.05.2001
(73) Patentinhaber: Henkel Kommanditgesellschaft auf Aktien, 40589 Düsseldorf-Holthausen (DE)
(72) Erfinder: HÖFFKES, Horst, D-40595 Düsseldorf (DE); NEUHAUS, Winifried, D-40822 Mettmann (DE); SCHUMANN, Klaus, D-40699 Erkrath (DE)
(86) Internationale Anmeldenummer: PCT/EP1999/004063
(87) Internationale Veröffentlichungsnummer: WO 1999/066890

(56) Entgegenhaltungen:
- WO-A-91/17739
- DE-A- 4 335 623
- DE-A- 4 335 628
- DE-A- 4 409 143
- DE-A- 19 717 282
- DE-A- 19 732 975
- US-A- 5 611 817

## Beschreibung

Die Erfindung betrifft Mittel zum Färben von Keratinfasern, insbesondere menschlichen Haaren, die spezielle Farbstoffvorprodukte vom Indol- oder Indolin-Typ sowie eine Komponente zur Intensivierung und/oder Nuancierung der Farbe enthalten, die Verwendung dieser Komponente zur Intensivierung und/oder Nuancierung von Färbungen sowie entsprechende Färbeverfahren.

Im Rahmen der Produkte, die zur kosmetischen Behandlung des menschlichen Körpers bereitgestellt werden, spielen Mittel zur Veränderung oder Nuancierung des Farbe des Kopfhaares eine herausragende Rolle. Sieht man von den Blondiermitteln, die eine oxidative Aufhellung der Haare durch Abbau der natürlichen Haarfarbstoffe bewirken, ab, so sind im Bereich der Haarfärbung seit längerer Zeit im wesentlichen zwei Typen von Haarfärbemitteln von Bedeutung:

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende Färbeergebnisse aus, können jedoch für bestimmte, eng umgrenzte Personenkreise auch mit Nachteilen behaftet sein. So können bestimmte Farbstoffvorprodukte bei sog. "Para-Allergikern" zu unerwünschten Hautirritationen führen. Weiterhin werden die Oxidationsfarbstoffe in der Regel mit Oxidationsmitteln, insbesondere Wasserstoffperoxid, ausgefärbt. Dies kann im Falle häufiger Anwendung bei Personen mit empfindlichen Haaren zu Beeinträchtigungen oder gar Schädigungen in der Haarstruktur führen, denen dann wieder mit speziellen Pflegeprodukten abgeholfen werden muß. Nicht zu unterschätzen ist auch die Anzahl der Personen, die im Rahmen der populären "Naturstoff/Chemie"-Diskussion die Verwendung chemischer Produkte aufgrund ihres persönlichen Standpunktes wo immer möglich vermeiden.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe mehr benötigen. Zu diesen Farbstoffen gehört beispielsweise das bereits aus dem Altertum zur Färbung von Körper und Haaren bekannte Henna.

Da beiden Färbeverfahren jedoch in den Augen vieler Verbrauchern der Hauch von "Künstlichem" mit seinen negativen Assoziationen anhaftet, hat in jüngster Zeit ein neuartiges Färbeverfahren große Beachtung gefunden. Bei diesem Verfahren werden Vorstufen des natürlichen Haarfarbstoffes Melanin auf das Haar aufgebracht und bilden in Rahmen oxidativer Prozesse im Haar somit praktisch naturidentische Farbstoffe aus. Ein solches Verfahren mit 5,6-Dihydroxyindolin als Farbstoffvorprodukt wurde in der EP-B1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß.

Unter den genannten Bedingungen lassen sich befriedigende Ergebnisse aber nur bei solchen Personen erzielen, die vor dem "Ergrauen" eine mittelblonde bis dunkelbraune Haarfarbe aufwiesen. Es hat daher nicht an Versuchen gefehlt, dieses neue

Färbeverfahren so zu modifizieren, daß auch Personen mit ursprünglich roter und insbesondere dunkler bis schwarzer Haarfarbe die ursprüngliche Haarfarbe wiedergegeben werden kann.

Eine Möglichkeit zur Erzielung dunklerer bis schwarzer Farbtöne, insbesondere solcher Farbtöne, die vom Fachmann als "matt" bezeichnet werden, ist Gegenstand der deutschen Patentanmeldung 197 32 975.6, auf die, insbesondere hinsichtlich des dort genannten Standes der Technik, ausdrücklich Bezug genommen wird. Die dort vorgeschlagene Lösung besteht in der Zugabe üblicher Kupplerkomponenten. Zwar kann die Ausfärbung auch unter alleiniger Verwendung von Luftsauerstoff erfolgen, jedoch wird dort bevorzugt der Einsatz mindestens eines weiteren Oxidationsmittels empfohlen.

Aufgrund der bereits oben erwähnten Vorbehalte vieler Verbraucher besteht jedoch nach wie vor der Bedarf nach einem Mittel, das bei Personen mit ergrautem Haar die natürliche Haarfarbe auch im Bereich dunkler bis schwarzer Haare wiederherstellt, ohne auf rein synthetische Farbstoffkomponenten sowie die Verwendung anderer Oxidationsmittel als Luftsauerstoff angewiesen zu sein.

Es wurde nun überraschenderweise gefunden, daß die genannte Aufgabe durch Anwendung eines Mittels gelöst werden kann, das neben den bekannten Farbstoffvorprodukten von Indol- oder Indolin-Typ mindestens eine Aminosäure oder ein Oligopeptid enthält.

Gegenstand der vorliegenden Erfindung sind daher Mittel zum Färben von Keratinfasern, insbesondere menschlichen Haaren, enthaltend ein Farbstoffvorprodukt, ausgewählt aus der Gruppe, die von Indolin-Derivaten und Indol-Derivaten gebildet wird, die weiterhin mindestens eine Aminosäure oder ein Oligopeptid enthalten.

Aminosäuren im Sinne der Erfindung sind solche Substanzen, die mindestens eine Aminogruppe und mindestens eine -COOH- oder -SO₃H-Gruppe aufweisen.

Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Ornithin und Histidin besonders bevorzugt.

Die Aminosäuren können den erfindungsgemäßen Mitteln bevorzugt in freier Form zugegeben werden. Es ist aber auch möglich, die Aminosäuren in Salzform einzusetzen. Bevorzugte Salze sind dann die Verbindungen mit Halogenwasserstoffsäuren, insbesondere die Hydrochloride und die Hydrobromide.

Eine ganz besonders bevorzugte Aminosäure ist Arginin, insbesondere in freier Form. aber auch als Hydrochlorid eingesetzt.

Selbstverständlich umfaßt die Erfindung auch solche Mittel, die zwei und mehr Aminosäuren oder Oligopeptide enthalten. Bevorzugt sind dabei Kombinationen von Arginin mit einer weiteren Aminosäure oder einem Oligopeptid.

Weiterhin können die Aminosäuren auch in Form von Oligopeptiden und Proteinhydrolysaten eingesetzt werden, wenn sichergestellt ist, daß die erforderlichen Mengen an Verbindungen, die der erfindungsgemäßen Definition der Aminosäuren entsprechen, darin enthalten sind. In diesem Zusammenhang wird auf die Offenbarung der DE-OS 22 15 303 verwiesen.

Die erfindungsgemäßen Mittel enthalten die Aminosäure bzw. das Oligopeptid bevorzugt in Mengen von 0,1 bis 10 Gew.-%, insbesondere 1 bis 4 Gew.-%, bezogen auf das gesamte Mittel.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d.h. auf pH-Werte im Bereich von etwa 5 bis 11, eingestellt. Zu diesem Zweck enthalten die Färbemittel Alkalisierungsmittel. üblicherweise Alkali- oder Erdalkalihydroxide, Ammoniak oder organische Amine.

Gemäß einer speziellen Ausführungsform der vorliegenden Erfindung dient die Aminosäure oder das Oligopeptid nicht nur der Generation der Ausfärbung, sondern übernimmt auch, zumindest teilweise, die Funktion des Alkalisierungsmittels. Im Rahmen dieser Ausführungsform werden daher bevorzugt solche Aminosäuren und Oligopeptide eingesetzt, deren 2,5 Gew.-%ige Lösungen in Wasser einen pH-Wert von 9 und größer aufweisen. Eine solche Aminosäure ist das bevorzugt eingesetzte Arginin. Im Rahmen dieser Ausführungsform wird das weitere Alkalisierungsmittel bevorzugt ausgewählt aus der Gruppe, die von Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxiden gebildet wird. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist im Rahmen dieser Ausführungsform bevorzugt.

Besonders vorteilhafte Eigenschaften wurden bei Mitteln gefunden, bei denen die Aminosäure oder das Oligopeptid und das weitere Alkalisierungsmittel in einem Verhältnis von 1:5 bis 5:1 vorlagen. Mengenverhältnisse von 1:2 bis 2:1 haben sich als besonders geeignet erwiesen.

Als weitere zwingende Komponente enthalten die erfindungsgemäßen Mittel ein Farbstoffvorprodukt von Indol- oder Indolin-Typ.

Erfindungsgemüß bevorzugt sind solche Indole und Indoline, die mindestens eine Hydroxy- oder Aminogruppe, bevorzugt als Substituent am Sechsring, aufweisen. Diese Gruppen können weitere Substituenten tragen, z.B. in Form einer Veretherung oder Veresterung der Hydroxygruppe oder eine Alkylierung der Aminogruppe.

Verbindungen mit zwei dieser Gruppen, insbesondere zwei Hydroxygruppen. von denen eine oder beide verethert oder verestert sein können, sind besonders bevorzugt.

Erfindungsgemäß besonders bevorzugte Farbstoffvorprodukte sind Derivate des Indolins, wie das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 4-, 6- und 7-Hydroxyindolin, 6-Aminoindolin und 4-Aminoindolin.

Ganz besonders bevorzugt sind Derivate des 5,6-Dihydroxyindolins der Formel (Ia), 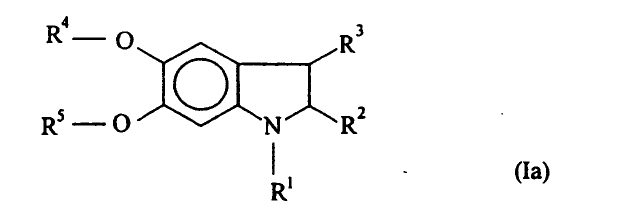 in der unabhängig voneinander
R¹ steht für Wasserstoff, eine C₁-C₄-Alkylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der R⁶ steht für eine C₁-C₄-Alkylgruppe, und
R⁵ steht für eine der unter R⁴ genannten Gruppen,
oder ein physiologisch verträgliches Salz dieser Verbindungen mit einer organischen oder anorganischen Säure.

Erfindungsgemäß bevorzugte Vertreter sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin. Der Grundkörper, das 5,6-Dihydroxyindolin, ist ganz besonders bevorzugt.

Erfindungsgemäß bevorzugte Indole sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 4-, 6- und 7-Hydroxyindol, 6-Aminoindol und 4-Aminoindol.

Besonders bevorzugt sind Derivate des 5,6-Dihydroxyindols der Formel (Ib), 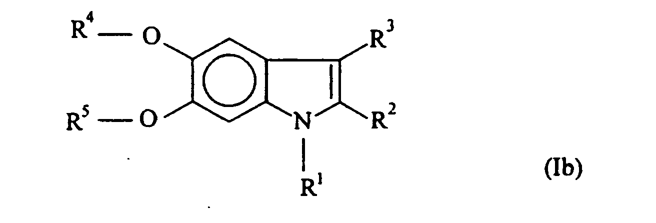 in der unabhängig voneinander
R¹ steht für Wasserstoff, eine C₁-C₄-Alkylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der R⁶ steht für eine C₁-C₄-Alkylgruppe, und
R⁵ steht für eine der unter R⁴ genannten Gruppen,
oder ein physiologisch verträgliches Salz dieser Verbindungen mit einer organischen oder anorganischen Säure.

Erfindungsgemäß bevorzugte Vertreter sind das 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol. Der Grundkörper, das 5,6-Dihydroxyindol, ist ganz besonders bevorzugt.

Die in den erfindungsgemäßen Mitteln enthaltenen Indolin- und Indol-Derivate können sowohl als freie Basen als auch in Form ihrer physiologisch verträglichen Salze mit anorganischen oder organischen Säuren, z.B. der Hydrochloride, der Sulfate und Hydrobromide, eingesetzt werden.

Die Indol- oder Indolin-Derivate sind in den erfindungsgemäßen Mitteln üblicherweise in Mengen von 0,05-10 Gew.-%, vorzugsweise 0,2-5 Gew.-% enthalten.

Selbstverständlich umfaßt die vorliegende Erfindung auch Mittel, die mehr als ein Indolin- oder Indol-Derivat oder Mischungen von Indolin- und Indol-Derivaten enthalten.

Gemäß einer besonders bevorzugten Ausführungsform enthalten die erfindungsgemäßen Mittel außer den genannten Indolen und Indolinen keine weiteren Farbstoffe oder Farbstoffvorprodukte.

Gleichwohl soll die Mitverwendung weiterer Farbstoffkomponenten oder Farbstoffvorprodukte aber nicht prinzipiell ausgeschlossen sein.

Im Rahmen der Ausführungsformen mit solchen weiteren Verbindungen sind folgende Substanzen bevorzugt:

Bevorzugte Entwickler-Komponenten:
p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-arnino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxyethylaminomethyl-4-amino-phenol, 4-Amino-2-((diethylamino)-methyl)-phenol, 4,4'-Di-aminodiphenylamin, 4-Amino-3-fluorphenol, 2-Aminomethyl-4-amino-phenol, 2-Hydroxymethyl-4-aminophenol, Bis-(2-hydroxy-5-aminophenyl)-methan, 1,4-Bis-(4-aminophenyl)-diazacycloheptan, 1,3-Bis-(N-(2-hydroxyethyl)-N-(4-aminophenylamino))-2-propanol, 4-Amino-2-(2-hydroxyethoxy)-phenol sowie 4,5-Diaminopyrazol-Derivate nach EP 0 740 741 bzw. WO 94/08970 wie z.B. 4,5-Diamino-1-(2'-hydroxyethyl)-pyrazol.

Besonders bevorzugte Entwickler-Komponenten:
p-Phenylendiamin, p-Toluylendiamin, p-Aminophenol, 1-(2'-Hydroxyethyl)-2,5-diaminobenzol, 4-Amino-3-methylphenol, 4-Amino-2-((diethylamino)-methyl)-phenol, 2,4,5,6-Tetraaminopyrimidin, 2-Hydroxy-4,5,6-triaminopyrimidin und 4-Hydroxy-2,5,6-triaminopyrimidin.

Bevorzugte Kuppler-Komponenten:
- m-Aminophenol und dessen Derivate wie beispielsweise 5-Amino-2-methylphenol, 5-(3-Hydroxypropylamino)-2-methylphenol, 3-Amino-2-chlor-6-methylphenol, 2-Hydroxy-4-aminophenoxyethanol, 3-Amino-6-methoxy-2-methylaminophenol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5- (2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-3,4-diaminopyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 3,4-Methylendioxyphenol, 1-Hydroxy-3,4-methylendioxybenzol, 1-Amino-3,4-methylendioxybenzol und 1-(2'-Hydroxyethyl)-amino-3,4-methylendioxybenzol,

Besonders bevorzugte Kuppler-Komponenten:
1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-diaminopyridin

Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol.
Bevorzugte direktziehende Farbstoffe sind auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel. Catechu, Sedre und Alkannawurzel.

Es ist dabei nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Sowohl die Oxidationsfarbstoffvorprodukte als auch die direktziehenden Farbstoffe sind in den erfindungsgemäßen Mitteln bevorzugt in Mengen von 0,01 bis 20 Gew.-%, vorzugsweise 0,5 bis 5 Gew.-%, jeweils bezogen auf das gesamte Mittel, enthalten.

Im Rahmen der Mittel, die weitere Farbstoffe oder Farbstoffvorprodukte enthalten, sind solche bevorzugt, die kein Oxidationsfarbstoffvorprodukt vom Entwickler-Typ enthalten. Im Rahmen dieser Ausführungsform der Erfindung enthalten die entsprechenden Mittel ein Oxidationsfarbstoffvorprodukt vom Kuppler-Typ sowie gewünschtenfalls noch direktziehende Farbstoffe.

Ebenfalls bevorzugt sind solche Mittel, die kein Oxidationsfarbstoffvorprodukt vom Kuppler-Typ enthalten. Diese Mittel sind bevorzugt auch frei von Oxidationsfarbstoffvorprodukten vom Entwickler-Typ, können aber einen direktziehenden Farbstoff, bevorzugt aus der Reihe der in der Natur vorkommenden Farbstoffe, enthalten.

Zur Herstellung der erfindungsgemäßen Färbemittel werden die oben genannten zwingenden und fakultativen Bestandteile in einen geeigneten wasserhaltigen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Die erfindungsgemäßen Färbemittel werden bevorzugt auf einen pH-Wert von 5 bis 11, insbesondere von 7 bis 10, eingestellt.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Anionische Tenside können dabei ganz besonders bevorzugt sein.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ether-, Amid- und Hydroxylgruppen sowie in der Regel auch Estergruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare und verzweigte Fettsäuren mit 8 bis 22 C-Atomen (Seifen),
- Ethercarbonsäuren der Formel R-O-(CH₂-CH₂O)_{X}-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(-CH₂-CH₂O)_{X}-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylen glykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungs produkte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammoniumglycinate. beispielsweise das Kokosalkyl-dimethylammoniumglycinat, N-Acylaminopropyl-N,N-dimethyl-ammoniumglycinate, beispielsweise das Kokosacylaminopropyl-dimethyl-ammonium-glycinat, und 2-Alkyl-3-carboxymethyl-3-hydroxyethylimidazoline mit jeweils 8 bis 18 C-Atomen in der Alkyl- oder Acylgruppe sowie das Kokosacyl-aminoethyl-hydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C8-C18-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C12-18-Acylsarcosin.

Nichtionische Tenside enthalten als hydrophile Gruppe z. B. eine Polyolgruppe, eine Polyalkylenglykolethergruppe oder eine Kombination aus Polyol- und Polyglykol-ethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C12-C22-Fettsäuremono- und -diester von Anlagerungsprodukten von 1 bis 30 Mol Ethylenoxid an Glycerin,
- C8-C22-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester,
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammonitunchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Coming 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane. Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methyl-hydroxyalkyl-dialkoyloxyalkyl-ammonium-methosulfate sowie die entsprechenden Produkte, die unter dem Warenzeichen Dehyquart® im Handel erhältlich sind.

Ein Beispiel für ein als kationisches Tensid einsetzbares quaternäres Zuckerderivat stellt das Handelsprodukt Glucquat® 100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen. Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-. Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes, wie beispielsweise α- und β-Hydroxycarbonsäuren
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft,
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Gemäß einer bevorzugten Ausführungsform erfolgt die Ausbildung der Färbung mit Luftsauerstoff als einzigem Oxidationsmittel.

Insbesondere in Fällen, in denen die Mittel zusätzlich Oxidationsfarbstoffvorprodukte vom Kuppler- oder Entwickler-Typ enthalten, soll jedoch die Mitverwendung eines chemischen Oxidationsmittels nicht prinzipiell ausgeschlossen sein. Dies gilt auch, wenn neben der Färbung ein Aufhelleffekt an menschlichem Haar gewünscht ist. Als Oxidationsmittel kommen dann insbesondere Wasserstoffperoxid oder dessen Anlagerungsprodukte an Harnstoff, Melamin oder Natriumborat in Frage. Weiterhin ist es möglich, die Oxidation mit Hilfe von Enzymen durchzuführen. Dabei können die Enzyme sowohl zur Erzeugung von oxidierenden Per-Verbindungen eingesetzt werden, als auch zur Verstärkung der Wirkung einer geringen Menge vorhandener Oxidationsmittel. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Unabhängig davon, welches der oben genannten Verfahren zur Anwendung des erfindungsgemäßen Mittels gewählt wird, kann die Ausbildung der Färbung dadurch unterstützt und gesteigert werden, daß dem Mittel bestimmte Metallionen zugesetzt werden. Solche Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinfluß werden.

Ein weiterer Gegenstand der Erfindung ist die Verwendung einer Aminosäure oder eines Oligopeptides zur Intensivierung und / oder Nuancierung der Ausfärbungen bei der Färbung keratinischer Fasern mit Mitteln, die als Farbstoffvorprodukte ein Indolin-Derivat oder ein Indol-Derivat enthalten.

Ein weiterer Gegenstand der Erfindung ist ein Verfahren zum Färben menschlicher Haare, bei dem eines der oben genannten Mittel auf das Haar aufgebracht wird und anschließend die Ausfärbung erfolgt. Dabei ist die Ausbildung der Farbe mittels Luftsauerstoff bevorzugt.

Gemäß einer besonderen Ausführungsform dieses Verfahrens erfolgt die Ausbildung der endgültigen Färbung durch mehrmaliges Aufbringen des Mittels und jeweils anschließender Luftoxidation. Das jeweilige Aufbringen des Mittels erfolgt dabei bevorzugt in Abständen, die zwischen etwa einem Tag und etwa zwei Wochen liegen. Dadurch können sehr gezielt spezielle Nuancen erhalten werden.

Die nachfolgenden Beispiele sollen den Erfindungsgegenstand näher erläutern.

### Beispiele

### 1. Ausfärbungen

Es wurden zunächst Färbemittel mit den in Tabelle 1 aufgeführten Zusammensetzungen hergestellt [alle Angaben sind, soweit nicht anders vermerkt, in g].

Die Ausfärbung erfolgte auf Haarsträhnen von ca. 5 cm Länge und ca. 0,5 g Gewicht. 1g des zu untersuchenden Mittels wurde auf das Haar aufgetragen. Nach 20minütiger Applikation (Luftoxidation) wurde das Mittel mit Wasser ausgespült und mit einem handelsüblichen Shampoo nachshampooniert. Die in der Tabelle 2 aufgeführten Ausfärbungen entsprechen den Verhältnissen nach eintägiger Lagerung der Haarsträhnen bei Raumtemperatur und üblichen Luftfeuchtigkeitsbedingungen von ca. 50 % relativer Feuchtigkeit.

**Tabelle 1:**

| Rezepturen | | | | | |
|---|---|---|---|---|---|
| Komponente | B1 | B2 | V1 | V2 | V3 |
| • Stenol® 1618 O¹ | 6,7 | 6,7 | 6,7 | 6,7 | 6,7 |
| • Lorol®techn² | 2,0 | 2,0 | 2,0 | 2,0 | 2,0 |
| • Eumulgin®B 2³ | 2,0 | 2,0 | 2,0 | 2,0 | 2,0 |
| • Ascorbinsäure | 0,2 | 0,2 | 0,2 | 0,2 | 0,2 |
| • Ammoniumsulfat | - | - | 1,0 | - | - |
| • 5,6-Dihydroxyindolinhydrobromid | 1,0 | - | 1,0 | 1,0 | - |
| • 5,6-Diacetoxyindol | - | 1,0 | - | - | 1,0 |
| • Kaliumhydroxid ad pH 9,5 | - | - | X | X | X |
| • Arginin (ad pH 9,5) | 3,0 | 3,0 | - | - | - |
| • Wasser | <-----ad 100 -----> | | | | |

| | | | | | |
|---|---|---|---|---|---|
| ¹ C₁₆₋₁₈-Fettalkohol (HENKEL) | | | | | |
| ² C₁₂₋₁₈-Fettalkohol (HENKEL) | | | | | |
| ³ Cetylstearylalkohol mit ca. 20 Mol EO (CTFA-Bezeichnung: Ceteareth-20) (HENKEL) | | | | | |

**Tabelle 2:**

| Ausfärbungen [Farbtiefe / Farbnuance] | | |
|---|---|---|
| Rezeptur | blondes Menschenhaar (Typ "naturweiß" (Kerling)) | graues Menschenhaar ( Typ "natural medium grey" (Klugmann # 6623) |
| B1 | mittelblond-dunkelblond / grau mit leichtem Blaustich | mittelbraun / mit leichtem Blaustich (mattes Mittelbraun) |
| | | |
| B2 | mittelblond / grau mit leichtem Blaustich | hellbraun / neutral, kein Blaustich erkennbar |
| | | |
| V1 | mittelblond / Naturton mit leichtem Rotstich | hellbraun / Naturton mit leichtem Rotstich |
| | | |
| V2 | hellblond-mittelblond / bläulich | dunkelblond / mit leichtem Blaustich |
| | | |
| V3 | hellblond / leichter Blaustich | dunkelblond / neutral, kein Blaustich erkennbar |

## Patentansprüche

1. Mittel zum Färben von Keratinfasern, insbesondere menschlichen Haaren, enthaltend ein Farbstoffvorprodukt, ausgewählt aus der Gruppe, die von Indolin-Derivaten und Indol-Derivaten gebildet wird, **dadurch gekennzeichnet, daß** das Mittel weiterhin mindestens eine Aminosäure oder ein Oligopeptid enthält.

2. Mittel nach Anspruch 1, **dadurch gekennzeichnet, daß** die Aminosäure oder das Oligopeptid als 2,5 Gew.-%ige Lösung in Wasser einen pH-Wert von größer als 9 aufweist.

3. Mittel nach Anspruch 1 oder 2, **dadurch gekennzeichnet, daß** es sich um eine α-Aminosäure handelt.

4. Mittel nach Anspruch 3, **dadurch gekennzeichnet, daß** die α-Aminosäure ausgewählt ist aus Arginin, Omithin, Lysin und Histidin.

5. Mittel nach Anspruch 4, **dadurch gekennzeichnet, daß** die α-Aminosäure Arginin ist.

6. Mittel nach einem der Ansprüche 1 bis 5, **dadurch gekennzeichnet, daß** das Farbstoffvorprodukt ein Indolin-Derivat ist.

7. Mittel nach Anspruch 6, **dadurch gekennzeichnet, daß** das Farbstoffvorprodukt ein Derivat des 5,6-Dihydroxyindolins der Formel (Ia) ist, 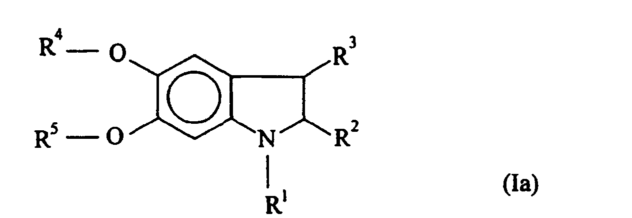 in der unabhängig voneinander
R¹ steht für Wasserstoff, eine C₁-C₄-Alkylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der R⁶ steht für eine C₁-C₄-Alkylgruppe, und
R⁵ steht für eine der unter R⁴ genannten Gruppen,
oder ein physiologisch verträgliches Salz dieser Verbindungen mit einer organischen oder anorganischen Säure.

8. Mittel nach Anspruch 7, **dadurch gekennzeichnet, daß** die Verbindung gemäß Formel (Ia) ausgewählt ist aus 5,6-Dihydroxyindolin, N-Methyl-5,6-dihydroxyindolin sowie deren physiologisch verträglichen Salzen.

9. Mittel nach einem der Ansprüche 1 bis 5, **dadurch gekennzeichnet, daß** das Farbstoffvorprodukt ein Indol-Derivat ist.

10. Mittel nach Anspruch 9, **dadurch gekennzeichnet, daß** das Farbstoffvorprodukt ein Derivat des 5,6-Dihydroxyindols der Formel (Ib) ist, 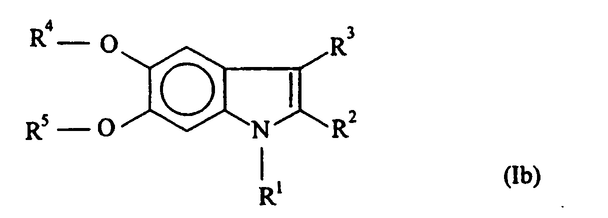 in der unabhängig voneinander
R¹ steht für Wasserstoff, eine C₁-C₄-Alkylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der R⁶ steht für eine C₁-C₄-Alkylgruppe, und
R⁵ steht für eine der unter R⁴ genannten Gruppen,
oder ein physiologisch verträgliches Salz dieser Verbindungen mit einer organischen oder anorganischen Säure.

11. Mittel nach Anspruch 10, **dadurch gekennzeichnet, daß** die Verbindung gemäß Formel (Ib) ausgewählt ist aus 5,6-Dihydroxyindol, N-Methyl-5,6-dihydroxyindol sowie deren physiologisch verträglichen Salzen.

12. Mittel nach einem der Ansprüche 1 bis 11, **dadurch gekennzeichnet, daß** es frei von Oxidationsfarbstoffvorprodukten vom Entwickler-Typ ist.

13. Mittel nach einem der Ansprüche 1 bis 12, **dadurch gekennzeichnet, daß** es frei von Oxidationsfarbstoffvorprodukten vom Kuppler-Typ ist.

14. Mittel nach einem der Ansprüche 1 bis 12, **dadurch gekennzeichnet, daß** die Kupplerkomponente ausgewählt ist aus 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-diaminopyridin und deren physiologisch verträglichen Salzen.

15. Mittel nach einem der Ansprüche 1 bis 14, **dadurch gekennzeichnet, daß** es einen direktziehenden Farbstoff enthält, der ausgewählt ist aus der Gruppe, die von HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16, Basic Brown 17, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol gebildet wird.

16. Mittel nach einem der Ansprüche 1 bis 15, **dadurch gekennzeichnet, daß** es einen direktziehenden Farbstoff enthält, der ausgewählt ist aus den in der Natur vorkommende Farbstoffen Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel.

17. Mittel nach einem der Ansprüche 1 bis 16, **dadurch gekennzeichnet, daß** das Indol- oder Indolin-Derivat in Mengen von 0,05-10 Gew.-%. vorzugsweise 0,2-5 Gew.-%, bezogen auf das gesamte Mittel, enthalten ist.

18. Mittel nach einem der Ansprüche 1 bis 17, **dadurch gekennzeichnet, daß** die Aminosäure oder das Oligopeptid in einer Menge von 0,1 - 10 %, bezogen auf das gesamte Mittel, enthalten ist.

19. Verwendung einer Aminosäure oder eines Oligopeptids zur Intensivierung und/oder Nuancierung der Ausfärbungen bei der Färbung keratinischer Fasern mit Mitteln, die als Farbstoff-vorprodukt ein Indolin-Derivat oder ein Indol-Derivat enthalten.

20. Verfahren zum Färben menschlicher Haare mit einem Mittel nach einem der Ansprüche 1 bis 18, **dadurch gekennzeichnet, daß** die Ausbildung der Farbe mit Luftsauerstoff erfolgt.

21. Verfahren nach Anspruch 20, **dadurch gekennzeichnet, daß** die Ausbildung der endgültigen Färbung durch mehrmaliges Aufbringen des Mittels und jeweils anschließende Luftoxidation erfolgt.

## Claims

1. A formulation for coloring keratin fibers, more particularly human hair, containing a dye precursor selected from the group consisting of indoline derivatives and indole derivatives, **characterized in that** it additionally contains at least one amino acid or one oligopeptide.

2. A formulation as claimed in claim 1, **characterized in that** the amino acid or the oligopeptide has a pH value above 9 in the form of a 2.5% by weight solution in water.

3. A formulation as claimed in claim 1 or 2, **characterized in that** the amino acid is an α-amino acid.

4. A formulation as claimed in claim 3, **characterized in that** the α-amino acid is selected from arginine, omithine, lysine and histidine.

5. A formulation as claimed in claim 4, **characterized in that** the α-amino acid is arginine.

6. A formulation as claimed in any of claims 1 to 5, **characterized in that** the dye precursor is an indoline derivative.

7. A formulation as claimed in claim 6, **characterized in that** the dye precursor is a derivative of 5,6-dihydroxyindoline corresponding to formula (la): 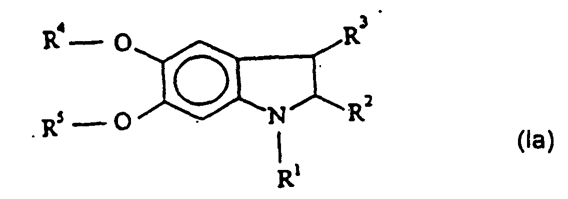 in which - independently of one another -
R¹ is hydrogen, a C₁₋₄ alkyl group or a C₁₋₄ hydroxyalkyl group,
R² is hydrogen or a -COOH group which may even be present as a salt with a physiologically compatible cation,
R³ is hydrogen or a C₁₋₄ alkyl group,
R⁴ is hydrogen, a C₁₋₄ alkyl group or a group -CO-R⁶ where R⁶ is a C₁₋₄ alkyl group,
R⁵ stands for one of the groups mentioned for R⁴,
or a physiologically compatible salt of these compounds with an organic or inorganic acid.

8. A formulation as claimed in claim 7, **characterized in that** the compound (la) is selected from 5,6-dihydroxyindoline, N-methyl-5,6-dihydroxyindoline and physiologically compatible salts thereof.

9. A formulation as claimed in any of claims 1 to 5, **characterized in that** the dye precursor is an indole derivative.

10. A formulation as claimed in claim 9, **characterized in that** the dye precursor is a derivative of 5,6-dihydroxyindole corresponding to formula (Ib): 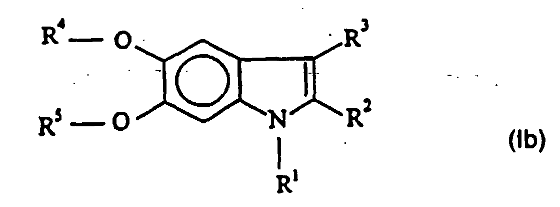 in which - independently of one another -
R¹ is hydrogen, a C₁₋₄ alkyl group or a C₁₋₄ hydroxyalkyl group,
R² is hydrogen or a -COOH group which may even be present as a salt with a physiologically compatiblecation,
R³ is hydrogen or a C₁₋₄ alkyl group,
R⁴ is hydrogen, a C₁₋₄ alkyl group or a group -CO-R⁶ where R⁶ is a C₁₋₄ alkyl group,
R⁵ stands for one of the groups mentioned for R⁴,
or a physiologically compatible salt of these compounds with an organic or inorganic acid.

11. A formulation as claimed in claim 10, **characterized in that** the compound (Ib) is selected from 5,6-dihydroxyindole, N-methyl-5,6-dihydroxyindole and physiologically compatible salts thereof.

12. A formulation as claimed in any of claims 1 to 11, **characterized in that** it is free from oxidation dye precursors of the primary intermediate type.

13. A formulation as claimed in any of claims 1 to 12, **characterized in that** it is free from oxidation dye precursors of the secondary intermediate type.

14. A formulation as claimed in any of claims 1 to 12, **characterized in that** the secondary intermediate is selected from the group consisting of 1-naphthol, 1,5-, 2,7- and 1,7-dihydroxynaphthalene, 3'-aminophenol, 5-amino-2-methylphenol, 2-amino-3-hydroxypyridine, resorcinol, 4-chlororesorcinol, 2-chloro-6-methyl-3-aminophenol, 2-methylresorcinol, 5-methylresorcinol, 2,5-dimethylresorcinol, 2,6-dihydroxy-3,4-diaminopyridine and physiologically compatible salts thereof.

15. A formulation as claimed in any of claims 1 to 14, **characterized in that** it contains a substantive dye selected from the group consisting of HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16, Basic Brown 17, 4-amino-2-nitrodiphenylamine-2'-carboxylic acid, 6-nitro-1,2,3,4-tetrahydroquinoxaline, hydroxyethyl-2-nitrotoluidine, picramic acid, 2-amino-6-chloro-4-nitrophenol, 4-ethylamino-3-nitrobenzoic acid and 2-chtoro-6-ethytamino-1-hydroxy-4-nitrobenzene.

16. A formulation as claimed in any of claims 1 to 15, **characterized in that** it contains a substantive dye selected from the group of naturally ocurring dyes consisting of henna red, henna neutral, henna black, camomile blossom, sandalwood, black tea, black alder bark, sage, logwood, madder root, catechu, sedre and alkanet.

17. A formulation as claimed in any of claims 1 to 16, **characterized in that** the indole or indoline derivative is present in quantities of 0.05 to 10% by weight and preferably in quantities of 0.2 to 5% by weight, based on the formulation as a whole.

18. A formulation as claimed in any of claims 1 to 17, **characterized in that** the amino acid or the oligopeptide is present in a quantity of 0.1 to 10% by weight, based on the formulation as a whole.

19. The use of an amino acid or oligopeptide for intensifying and/or shading the colors in the coloring of keratin fibers with formulations containing an indoline derivative or an indole derivative as dye precursors.

20. A process for coloring human hair with the formulation claimed in any of claims 1 to 18, **characterized in that** the color is developed with atmospheric oxygen.

21. A process as claimed in claim 20, **characterized in that** the final color is developed by repeated application of the formulation, followed after each application by oxidation with air.

## Revendications

1. Agent pour colorer les fibres de kératine, en particulier les cheveux humains, contenant un précurseur de colorant, choisi dans le groupe qui est formé par les dérivés d'indoline et les dérivés d'indole, **caractérisé en ce que** l'agent contient en outre au moins un acide aminé ou un oligopeptide.

2. Agent selon la revendication 1, **caractérisé en ce que** l'acide aminé ou l'oligopeptide sous forme de solution aqueuse à 2,5% présente une valeur de pH supérieure à 9.

3. Agent selon la revendication 1 ou 2, **caractérisé en ce qu'**il s'agit d'un acide α-aminé.

4. Agent selon la revendication 3, **caractérisé en ce que** l'acide α-aminé est choisi parmi l'arginine, l'ornithine, la lysine, et l'histidine.

5. Agent selon la revendication 4, **caractérisé en ce que** l'acide α-aminé est l'arginine.

6. Agent selon l'une quelconque des revendications 1 à 5, **caractérisé en ce que** le précurseur de colorant est un dérivé de l'indoline.

7. Agent selon la revendication 6, **caractérisé en ce que** le précurseur de colorant est un dérivé de la 5,6-dihydroxyindoline de formule (la), 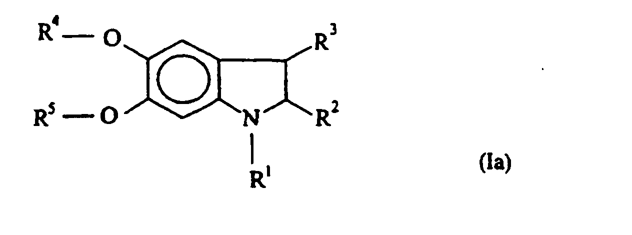 dans laquelle, indépendamment l'un de l'autre,
R¹ représente un atome d'hydrogène, un groupe alkyle en C₁ à C₄ ou un groupe hydroxyalkyle en C₁ à C₄,
R² représente un atome d'hydrogène, ou un groupe -COOH, le groupe -COOH pouvant également exister sous forme de sel avec un cation acceptable sur le plan physiologique,
R³ représente un atome d'hydrogène ou un groupe alkyle en C₁ à C₄.
R⁴ représente un atome d'hydrogène, un groupe alkyle en C₁ à C₄ ou un groupe -CO-R⁶, dans lequel R⁶ représente un groupe alkyle en C₁ à C₄, et
R⁵ représente un des groupes cités sous R⁴,
ou un sel acceptable sur le plan physiologique de ces composés, avec un acide organique ou inorganique.

8. Agent selon la revendication 7, **caractérisé en ce que** le composé selon la formule (la) est choisi parmi la 5,6-dihydroxyindoline, la N-méthyl-5,6-dihydroxyindoline, ainsi que parmi leurs sels acceptables sur le plan physiologique.

9. Agent selon l'une quelconque des revendications 1 à 5, **caractérisé en ce que** le précurseur de colorant est un dérivé de l'indole.

10. Agent selon la revendication 9, **caractérisé en ce que** le précurseur de colorant est un dérivé du 5,6-hydroxyindole, de formule (Ib), 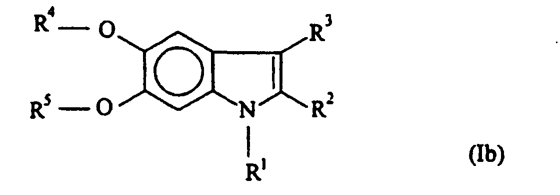 dans laquelle, indépendamment l'un de l'autre,
R¹ représente un atome d'hydrogène, un groupe alkyle en C₁ à C₄ ou un groupe hydroxyalkyle en C₁ à C₄,
R² représente un atome d'hydrogène, ou un groupe -COOH, le groupe -COOH pouvant également exister sous forme de sel avec un cation acceptable sur le plan physiologique,
R³ représente un atome d'hydrogène ou un groupe alkyle en C₁ à C₄.
R⁴ représente un atome d'hydrogène, un groupe alkyle en C₁ à C₄ ou un groupe -CO-R⁶, dans lequel R⁶ représente un groupe alkyle en C₁ à C₄, et
R⁵ représente un des groupes cités sous R⁴,
ou un sel acceptable sur le plan physiologique de ces composés, avec un acide organique ou inorganique.

11. Agent selon la revendication 10, **caractérisé en ce que** le composé selon la formule (Ib) est choisi parmi le 5,6-dihydroxyindole, le N-méthyl-5,6-dihydroxyindole, ainsi que parmi leurs sels, acceptables sur le plan physiologique.

12. Agent selon l'une quelconque des revendications 1 à 11, **caractérisé en ce qu'**il est exempt de précurseur de colorant par oxydation du type révélateur.

13. Agent selon l'une quelconque des revendications 1 à 12, **caractérisé en ce qu'**il est exempt de précurseur de colorant par oxydation du type agent de couplage.

14. Agent selon l'une quelconque des revendications 1 à 12, **caractérisé en ce que** le composant agent de couplage est choisi parmi le 1-naphtol, le 1,5-, 2,7- et 1,7-dihydroxynaphtalène, le 3-aminophénol, le 5-amino-2-méthylphénol, la 2-amino-3- hydroxypyridingela résorcine, la 4-chlororésorcine, le 2-chloro-6-méthyl-3-aminophénol, la 2-méthylrésorcine, la 5-méthylrésorcine, la 2,5-diméthylrésorcine, la 2,6-dihydroxy-3,4-diaminopyridine, et leurs sels acceptables sur le plan physiologique.

15. Agent selon l'une quelconque des revendications 1 à 14, **caractérisé en ce qu'**il contient un colorant montant directement sur la fibre, qui est choisi dans le groupe formé par HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16, Basic Brown 17, l'acide 4-amino-2-nitrodiphénylamin-2'-carboxylique, la 6-nitro-1,2,3,4-tétrahydro-quinoxaline, la hydroxyéthyl-2-nitro-toluidine, l'acide picraminique, le 2-amino-6-chloro-4-nitrophénol, l'acide 4-éthylamino-3-nitrobenzoïque, et le 2-chloro-6-éthylamino-1-hydroxy-4-nitrobenzène.

16. Agent selon l'une quelconque des revendications 1 à 15, **caractérisé en ce qu'**il contient un colorant montant directement sur la fibre, qui est choisi parmi les colorants existant dans la nature, le henné rouge, le henné neutre, le henné noir, les fleurs de camomille, le bois de santal, le thé noir, l'écorce de bourdaine, la sauge, le bois de campêche, la racine de garance, le cachou, le cèdre, et la racine d'orcanette.

17. Agent selon l'une quelconque des revendications 1 à 16, **caractérisé en ce que** le dérivé de l'indole ou le dérivé de l'indoline est contenu en des quantités de 0,05 à 10% en poids, de préférence de 0,2 à 5% en poids, par rapport à l'agent entier.

18. Agent selon l'une quelconque des revendications 1 à 17, **caractérisé en ce que** l'acide aminé ou l'oligopeptide est contenu en des quantités de 0,1 à 10%, par rapport à l'agent entier.

19. Utilisation d'un acide aminé ou d'un oligopeptide pour l'intensification et/ou le nuançage des colorations lors de la coloration de fibres kératiniques avec des agents qui contiennent un dérivé de l'indoline ou un dérivé de l'indole, en tant que précurseur de colorant.

20. Procédé pour colorer les cheveux humains avec un agent selon l'une quelconque des revendications 1 à 18, **caractérisé en ce que** la formation de la couleur se réalise avec l'oxygène de l'air.

21. Procédé selon la revendication 20, **caractérisé en ce que** la formation de la coloration définitive se réalise en appliquant plusieurs fois l'agent et à chaque fois suivi par une oxydation par l'air.
